(19) Europäisches Patentamt
European Patent Office
Office européen des brevets

(11) Publication number: 0 102 140
A1

(12) EUROPEAN PATENT APPLICATION

(21) Application number: 83302464.9

(22) Date of filing: 29.04.83

(51) Int. Cl.³: C 07 C 85/06

(30) Priority: 12.05.82 US 377291

(43) Date of publication of application: 07.03.84 Bulletin 84/10

(84) Designated Contracting States: BE CH DE FR GB IT LI NL

(71) Applicant: THE PROCTER & GAMBLE COMPANY
301 East Sixth Street
Cincinnati Ohio 45201(US)

(72) Inventor: Rieke, Ross Dale
61 Carpenter's Ridge
Cincinnati Ohio 45241(US)

(74) Representative: Gibson, Tony Nicholas et al,
Procter & Gamble (NTC) Limited Whitley Road
Longbenton Newcastle upon Tyne NE12 9TS(GB)

# (54) Catalyzed preparation of amines from alkyl sulfates.

(57) An improved process for making $C_6$ to $C_{22}$ alkyl amines by the reaction of a $C_6$ to $C_{22}$ alkyl sulfate with ammonia or primary and secondary alkyl amines and strong base, the improvement comprising conduct of the reaction in the presence of an effective amount of a catalyst ion selected from $R_3SO_3^-$ (wherein $R_3$ is $C_1$ to $C_4$ alkyl, phenyl, or $C_1$ to $C_3$ alkyl-substituted phenyl), $I^-$, $Br^-$, $NO_3^-$, $Cl^-$ and $F^-$.

EP 0 102 140 A1

0102140

# CATALYZED PREPARATION OF AMINES FROM ALKYL SULFATES

Ross D. Rieke

## FIELD OF THE INVENTION

The invention relates to the production of long chain amines by reaction of ammonia, primary amines or secondary amines with long chain alkyl sulfates.

## BACKGROUND

U.S. Pat. No. 3,378,588, issued to Shapiro, April 16, 1968, discloses a process for preparation of tertiary amines from alkyl sulfate wherein a $C_6$ to $C_{22}$ alkyl sulfate is reacted with a secondary amine and an alkali metal hydroxide under pressure and at a temperature of from 100°C to about 200°C. U.S. Pat. No. 3,379,763, issued to Shapiro, April 23, 1968, discloses the reaction of $C_6$ to $C_{22}$ alkyl sulfates with alkali metal hydroxide and ammonia or primary amines under pressure to about 150°C to about 200°C to produce, respectively, primary and secondary amines containing the alkyl chain from the alkyl sulfate. A reaction time of from about 4 to about 8 hours is taught in both patents. Both patents are incorporated by reference herein.

The object of the present invention is to improve upon the process of U.S. Pat. Nos. 3,378,588 and 3,379,763 by catalyzing the reaction to reduce reaction time.

## SUMMARY OF THE INVENTION

An improved process for preparing an amine having a $C_6$ to $C_{22}$ alkyl group, comprising the step of

heating, in a closed system under pressure, a $C_6$ to $C_{22}$ alkyl sulfate, a nitrogen compound selected from ammonia and primary and secondary alkyl and hydroxyalkyl amines, having from 1 to 3 carbon atoms in their alkyl or hydroxyalkyl groups, and a strong base at a temperature of from 100°C to 250°C, the improvement comprising conducting said process in the presence of an effective amount of a catalyst selected from

$R_3SO_3^-$, $I^-$, $Br^-$, $NO_3^-$, $Cl^-$ and $F^-$

wherein $R_3$ is $C_1$ to $C_4$ alkyl, phenyl, or $C_1$ to $C_3$ alkyl-substituted phenyl.

## DETAILED DESCRIPTION OF THE INVENTION

In accordance with the present invention it has been found that the reaction of $C_6$ to $C_{22}$ alkyl sulfate, strong base and ammonia or primary and secondary amines to produce the corresponding primary, secondary or tertiary amines can be appreciably accelerated by conducting said reaction in the presence of a catalytic amount of an ion selected from $R_3SO_3^-$ (wherein $R_3$ is $C_1$ to $C_4$ alkyl, phenyl, or $C_1$ to $C_3$ alkyl-substituted phenyl), $I^-$, $Br^-$, $NO_3^-$, $Cl^-$ and $F^-$.

Amines containing the $C_6$ to $C_{22}$ alkyl group are useful as corrosion inhibitors and fabric conditioning agents and as intermediates in the preparation of germicides and surfactants.

The catalyzed process of the present invention will be illustrated by the reaction of alkyl sulfate with secondary amines to produce tertiary amines, however, it is to be understood that the analogous catalyzed reactions of alkyl sulfates with ammonia and primary amines to produce, respectively, the corresponding primary and secondary amines, are included within the broad scope of the invention.

The reaction of alkyl sulfate, secondary amine and strong base can be described as follows:

$$RSO_4Na + R_1R_2NH + NaOH \longrightarrow RNR_1R_2 + Na_2SO_4 + H_2O$$

wherein R is a $C_6$ to $C_{22}$ alkyl group, and $R_1$ and $R_2$ are lower alkyl or hydroxyalkyl groups containing from 1 to 4 carbon atoms.

Although the scope of the present invention is not to be limited by any theory, it is believed that when the reaction is conducted in the presence of a source of the aforedescribed catalyst ions (e.g., $I^-$) the following reaction sequence occurs (assume the strong base is NaOH, the alkyl sulfate is a sodium salt and the source of catalyst ion is NaI):

1. $RSO_4Na + NaI \longrightarrow RI + Na_2SO_4$

2. $RI + R_1R_2NH + NaOH \longrightarrow RNR_1R_2 + NaI + H_2O$

The NaI regenerated in Step 2 then reacts with additional $RSO_4Na$ and the reaction sequence is repeated. It is believed that the faster reaction rate achieved when iodide ion is used is due to the fact that it is much easier for the secondary amine to displace the $I^-$ group from RI than to displace the $SO_4M^-$ group from $RSO_4M$. The $R_3SO_3^-$, $Br^-$, $NO_3^-$, $Cl^-$ and $F^-$ groups are also easier to displace than $SO_4M^-$, therefore these ions also function as catalysts in the process herein.

The alkyl sulfate reactants in the process of the present invention have the formula

$$ROSO_3M$$

wherein R is an alkyl radical having from about 6 to about 22 carbon atoms and M is a cation selected from hydrogen and metal cations. Preferred cations are

hydrogen and the alkali (e.g., sodium and potassium) and alkaline earth (e.g., calcium and magnesium) metals. The most preferred cations are the alkali metals, particularly sodium.

The alkyl sulfates are commercially available materials and are usually prepared by the reaction of alkanols with oleum, sulfur trioxide or chlorosulfonic acid, followed by neutralization with a base such as caustic soda or caustic potash. The alkanols can be of natural or synthetic origin. Typical alkyl sulfates are the sulfates of octanol, dodecanol, tetradecanol, octadecanol, etc. Commercially available alkyl sulfates often contain mixed alkyl groups, such as, for example, alkyl sulfates made from the alkanols prepared by the reduction of the fatty acids derived from natural fats and oils such as coconut oil and tallow. Preferred alkyl sulfates for use in the process herein are those having alkyl groups of from about 8 to about 18 carbon atoms and mixtures thereof.

The nitrogen compounds used in the process herein are ammonia ($NH_3$) and primary and secondary amines of the formulas $R_1NH_2$ and $R_1R_2NH$, wherein $R_1$ and $R_2$ can be the same or different from each other and are selected from alkyl and hydroxyalkyl groups having from 1 to about 4 carbon atoms, preferably 1 to 2 carbon atoms. The primary and secondary amines are typically made by reacting ammonia with the appropriate alkyl halide. Typical primary and secondary amines for use herein include methylamine, ethylamine, butylamine, dimethylamine, diethylamine, dipropylamine, dibutylamine, methylethylamine, ethylbutylamine, di(2-hydroxyethyl)-amine, mono(2-hydroxyethyl)-amine, monoisopropanolamine and diisopropanolamine.

Any strong base can be used in the process herein. Preferably the base will be an alkali metal

hydroxide such as caustic soda or caustic potash. The most preferred base is caustic soda. The amount of base used should be stoichiometrically equivalent to the amount of hydrogen ion released by the amine, and also should be sufficient to neutralize the alkyl sulfate if an acid alkyl sulfate is used instead of a salt.

The catalytic ions of the process herein are selected from $R_3SO_3^-$ (wherein $R_3$ is $C_1$ to $C_4$ alkyl, phenyl, or $C_1$ to $C_3$ alkyl-substituted phenyl), $I^-$, $Br^-$, $NO_3^-$, $Cl^-$ and $F^-$. Examples of $R_3SO_3^-$ are methylsulfonate, propylsulfonate, phenylsulfonate, 4-methylphenylsulfonate, 3,5-diethylphenylsulfonate and 4-isopropylphenylsulfonate. The preferred catalyst ions are $I^-$ and $Br^-$. The catalyst ions herein are added to the process in the form of the acids (e.g., HI, HBr, etc.) or in the form of the corresponding salts (e.g., NaI, KBr, etc.) The salts are preferred, particularly the alkali metal salts, and most particularly the sodium salts. The most preferred catalyst is sodium iodide.

The reaction herein is carried out in a closed system under pressure. The pressure should generally be within the range of from 200 lbs./sq.in. to 1000 lbs./sq.in. (14.1 to 70.3 kg./sq.cm.). The preferred pressure is from 200 to 500 lbs./sq.in. (14.1 to 35.1 kg./sq.cm.). Reaction completion with high yield ( 95%) of tertiary amine can be completed in one to two hours. The reaction temperature should be within the range of from 100°C to 250°C. When the reactant is ammonia, the preferred temperature range is from 200°C to 250°C. When the reactant is a primary amine, the preferred temperature is from 150°C to 200°C. When the reactant is a secondary amine, the preferred temperature is from

100°C to 200°C, most preferably from 130°C to 160°C.

The amount of catalyst used should be an amount which is effective to increase the rate of reaction. Typically this will be from 5 to 100 molar percent of the amount of alkyl sulfate used. The reaction is preferably conducted in the presence of an inert solvent. Water is a preferred solvent.

The invention will be illustrated by the following example.

EXAMPLE 1

To a one liter stirred autoclave, 125 grams of sodium dodecyl sulfate (0.434 moles), 17.4 grams of sodium hydroxide (0.434 moles), 250 grams of water (as solvent, 13.88 moles), 280 grams dimethylamine (6.22 moles), and 32 grams sodium iodide (0.21 moles) is added and heated to a temperature of 154.4°C and a pressure of 500 psi (35.1 kg./sq.cm.). After 30 minutes of reaction time, conversion is 67.3 percent to a product that is 98.2% dimethyldodecylamine. After one hour of reaction time, the conversion is approximately 80 percent with the corresponding purity of 98.2% dimethyldodecylamine.

The above reaction is run with the quantities of materials charged to the autoclave reduced to three-fifths of the amounts recited above and without the addition of sodium iodide. A conversion of 50% is obtained after 30 minutes and a conversion of 66.7% is obtained after one hour.

CLAIMS

1. A process for preparing amines containing a $C_6$-$C_{22}$ alkyl group, in which a $C_6$ to $C_{22}$ alkyl sulfate, a strong base and a nitrogen compound selected from ammonia and primary and secondary alkyl and hydroxyalkyl amines containing from 1 to 4 carbon atoms in their alkyl or hydroxyalkyl groups are reacted at a temperature of from 100°C to 250°C, in a closed system under pressure, characterised in that the process is carried out in the presence of an effective amount of a catalyst selected from $R_3SO_3^-$, $I^-$, $Br^-$, $NO_3^-$, $Cl^-$ and $F^-$ wherein $R_3$ is $C_1$ to $C_4$ alkyl, phenyl or $C_1$ to $C_3$ alkyl-substituted phenyl.

2. The process of Claim 1 wherein the pressure is from 14.1 to 70.3 kg/sq cm.

3. The process of Claim 2 wherein the catalyst is selected from $I^-$ and $Br^-$.

4. The process of Claim 3 wherein when the nitrogen compound is ammonia, the temperature is from 200°C to 250°C, when the nitrogen compound is a primary amine the temperature is from 150°C to 200°C and when the nitrogen compound is a secondary amine the temperature is from 100°C to 200°C.

5. The process of Claim 4 wherein the nitrogen compound is a secondary amine, the temperature is from 130°C to 160°C and the pressure is from 14.1 to 35.1 kg/sq cm.

6. The process of Claims 1 to 5 wherein the amount of catalyst is from 5 to 100 mole percent of the amount of alkyl sulfate.

7. The process of Claim 6 wherein the alkyl sulfate is selected from $C_8$ to $C_{18}$ alkyl sulfates and mixtures thereof, the amine is dimethylamine, the strong base is caustic soda and the catalyst is $I^-$.